(19) Europäisches Patentamt
European Patent Office
Office européen des brevets

(11) Veröffentlichungsnummer : **0 019 712**
**B1**

(12) **EUROPÄISCHE PATENTSCHRIFT**

(45) Veröffentlichungstag der Patentschrift : **15.12.82**

(51) Int. Cl.³ : **C 07 C 85/11**, C 07 C143/62

(21) Anmeldenummer : **80101979.5**

(22) Anmeldetag : **14.04.80**

(54) **Verfahren zur partiellen Reduktion von polynitrierten Diarylverbindungen.**

(30) Priorität : **26.04.79 DE 2916815**

(43) Veröffentlichungstag der Anmeldung : **10.12.80 (Patentblatt 80/25)**

(45) Bekanntmachung des Hinweises auf die Patenterteilung : **15.12.82 Patentblatt 82/50**

(84) Benannte Vertragsstaaten : **CH DE FR GB LI**

(56) Entgegenhaltungen :
**LU A 50 216**
**CHEMICAL ABSTRACTS, Band 86, Nr. 21, 23. Mai 1977, Zusammenfassung Nr. 155368a, Seite 444, Columbus, Ohio, US**

(73) Patentinhaber : **BAYER AG**
**Zentralbereich Patente, Marken und Lizenzen**
**D-5090 Leverkusen 1, Bayerwerk (DE)**

(72) Erfinder : **Linhart, Karl, Dr.**
**Heymannstrasse 65**
**D-5090 Leverkusen 1 (DE)**
Erfinder : **Pawellek, Dieter, Dr.**
**Elisabeth-Langgässer-Strasse 38**
**D-5090 Leverkusen (DE)**
Erfinder : **Gleinig, Harald, Dr.**
**Eichholzer Weg 100**
**D-5068 Odenthal-Neschen (DE)**

**EP 0 019 712 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur partiellen Reduktion von polynitrierten Diarylverbindungen

Die vorliegende Erfindung betrifft ein Verfahren zur partiellen Reduktion von polynitrierten Diarylverbindungen der allgemeinen Formel

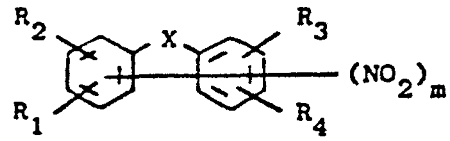

(I)

worin
$X = -CH = CH-$,
$R_1$, $R_2$, $R_3$, $R_4$ unabhängig voneinander = Wasserstoff, $C_1$-$C_4$-Alkyl, Halogen, Sulfonsäuregruppen,
m = 2-10,
zu Nitroaminodiarylverbindungen der allgemeinen Formel

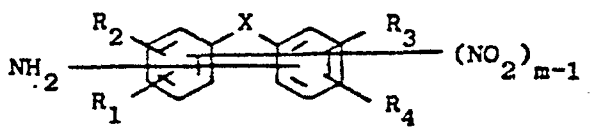

(II)

in wäßriger oder organischer Lösung oder Suspension mit Sulfiden oder Sulfidionen abgebenden Substanzen, dadurch gekennzeichnet, daß man pro zu reduzierender Nitrogruppe 1-2 Mol, vorzugsweise äquivalente Mengen, d.h. 1,5 Mol Sulfid verwendet und bei erhöhter Temperatur in Gegenwart von 0,5-10 Mol Ammoniak pro zu reduzierender Nitrogruppe arbeitet.

Als polynitrierte Diarylverbindungen seien im einzelnen beispielsweise genannt:
4,4'-Dinitro-stilben-2,2'-disulfonsäure.

Die im BIOS Final Report 1153, 197 (1948) gemachten Angaben über die partielle Reduktion der 4,4'-Dinitrostilben-2,2'-disulfonsäure lassen sich bei einer Nacharbeitung nicht bestätigen. Ein Molverhältnis von 0,68 Mol Schwefelwasserstoff pro Mol Nitrogruppe kann schon aus stöchiometrischen Gründen nicht im gewünschten Sinne ausreichen. Insbesondere bei den dort angeführten Temperaturen von 38 bis 43 °C erzielt man weder einen hohen Umsatz noch eine hohe Selektivität. Die Untersuchung des anfallenden Reaktionsgemisches zeigt deutlich, daß die im BIOS angegebene Vorschrift nicht zum gewünschten Erfolg führt. Das postulierte 4-Nitro-4'-amino-stilben-2,2'-disulfonsäure ist in nur 43 % entstanden. Es bleiben etwa 53,5 % nicht reduziertes Ausgangsmaterial zurück. Der Rest ist zu 4,4'-Diamino-stilben-2,2'-disulfonsäure reduziert worden.

Für die Reduktion sind die verschiedensten Sulfidverbindungen geeignet, z.B. Natriumhydrogensulfid, weiterhin Alkali- und Erdalkalimetallsulfide sowie Ammoniumsulfide.

Die Wahl der Temperatur kann in weiten Bereichen erfolgen. Vorteilhafterweise arbeitet man im Bereich von 30-110 °C, insbesondere bei 75-85 °C. Die Reduktionen verlaufen bei optimalen Temperaturen so rasch, daß sie auch kontinuierlich durchgeführt werden können.

Die Möglichkeit einer technischen Durchführung des Verfahrens sei am folgenden Beispiel der partiellen Reduktion von 4,4'-Dinitro-stilben-2,2'-disulfonsäure zu 4-Nitro-4'-amino-stilben-2,2'-disulfonsäure veranschaulicht:

Die wäßrige Lösung des Dinatriumsalzes der 4,4'-Dinitro-stilben-2,2'-disulfonsäure wird unter Zusatz von Ammoniak bei 75-85 °C mit äquivalenter Menge — bezogen auf die zu reduzierende Nitrogruppe — Natriumhydrogensulfidlösung versetzt. Der Verlauf der Reduktion läßt sich nach bekannten analytischen Methoden verfolgen. Nach beendeter partieller Reduktion wird die entstandene 4-Nitro-4'-amino-stilben-2,2'-disulfonsäure als Dinatriumsalz oder als inneres Salz isoliert. Die Verunreinigung an 4,4'-Diamino-stilben-2,2'-disulfonsäure beträgt weniger als 1 %, der Restgehalt an 4,4'-Dinitro-stilben-2,2'-disulfonsäure liegt unter 0,1 %. 4-Nitro-4'-amino-stilben-2,2'-disulfonsäure ist ein bekanntes Zwischenprodukt für Farbstoffe.

Das erfindungsgemäße Verfahren bei dem in Gegenwart von Ammoniak gearbeitet wird, liefert — wie die Nacharbeitung der Literaturangaben ergab — in einem einfacheren Verfahrensablauf überraschenderweise ein reineres Produkt in höherer Ausbeute als das Verfahren das in Chemical Abstracts, Vol. 86, 1977, 155 368 a beschrieben wird.

## Beispiel 1

(Kontrollversuch nach BIOS Final Report 1153, 197 (1948) I.G. Farben über die partielle Reduktion von 4,4'-Dinitro-stilben-2,2'-disulfonsäure).

Es werden 996 g einer 43,2 gew.-%igen Produktpaste, enthaltend 430 g (1,0 Mol) 4,4'-Dinitro-stil-

ben-2,2'-disulfonsäure, mit Wasser zu einer 22,8 gew.-%igen Suspension verdünnt. Bei Erwärmen auf vorschriftsmäßig 55 °C tritt keine Lösung ein. Auch bei Rückfluß liegt eine Suspension vor. In die gerührte Suspension läßt man 23 g (0,68 Mol) Schwefelwasserstoff, vorliegend als 38,1 g Natriumhydrogensulfid, in 163,4 g = 148,5 ml einer 23,3 gew.-%igen Natriumhydrogensulfidlösung (D : 1,1) zulaufen, wobei auf 38 °C fällt. Danach gibt man 200 ml konz. Salzsäure hinzu und bläst die stark saure Lösung 2 Stunden mit Luft aus, um das freigesetzte Schwefeldioxid zu entfernen. Man isoliert.

Nach dem Analysenergebnis liegen etwa 53,5 % nicht umgesetztes Ausgangsmaterial und etwa 3,5 % 4,4'-Diamino-stilben-2,2'-disulfonsäure neben 43 % 4-Nitro-4'-amino-stilben-2,2'-disulfonsäure vor.

Bei weiterer Zugabe von 148,5 ml einer 23,3 gew.-%igen Natriumhydrogensulfidlösung setzt sich der jetzt klar vorliegende Ansatz aus 16,7 % 4,4'-Dinitro-, 56,8 % 4-Nitro-4'-amino-stilben- und 26,5 % 4,4'-Diamino-stilben-2,2'-disulfonsäure zusammen.

Beispiel 2

Es werden 906,4 g einer 52,3 gew.-%igen Produktpaste, enthaltend 474 g (1 Mol) 4,4'-Dinitro-stilben-2,2'-disulfonsäure-dinatriumsalz, unter Zusatz von 1.000 ml Wasser und 336 ml = 306 g 25 gew.-%iger Ammoniumhydroxidlösung (D : 0,9), enthatend 76,5 g (4,5 Mol) Ammoniak, auf 75-85 °C erhitzt, wobei eine dunkelrote, klare 21,3 gew.-%ige Lösung resultiert, bezogen auf Dinitrostilbendisulfonsäure. Innerhalb 1 Stunde dosiert man eine Mischung von 244 ml einer 34,4 vol.-%igen Natriumhydrogensulfidlösung, enthaltend 84 g (1,5 Mol) Natriumhydrogensulfid, und 37 ml = 34 g 25 gew.-%ige Ammoniumhydroxidlösung, enthaltend 8,5 g (0,5 Mol) Ammoniak, hinzu. Die Analyse der Reaktionsmischung zeigt deutlich ein selektives und praktisch quantitatives Wegreduzieren einer Nitrogruppe im Molekül. Man säuert mit konz. Salzsäure bis zu einem pH 3,9 an. Nach Abkühlen auf Raumtemperatur saugt man die rote Paste ab. Nach der Analyse sind die 1.310 g 30,5 %ig, d.h. es liegen 399,6 g Produkt vor, was einer Ausbeute von 99,8 % d. Th. entspricht. Bezogen auf 100 g 100 %ige Ware enthält die 4-Nitro-4'-amino-stilben-2,2'-disulfonsäure lediglich 1 g 4,4'-Diamino-stilben-2,2'-disulfonsäure und weniger als 0,1 % 4,4'-Dinitro-stilben-2,2'-disulfonsäure.

**Ansprüche**

1. Verfahren zur partiellen Reduktion von polynitrierten Diarylverbindungen der Formel

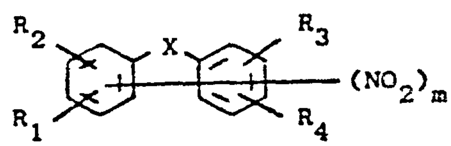

worin

X = —CH = CH—

$R_1$-$R_4$ unabhängig voneinander = Wasserstoff, $C_1$-$C_4$-Alkyl, Halogen, Sulfonsäuregruppen

m = 2-10

mit Sulfiden oder Sulfidionen abgebenden Substanzen bei erhöhter Temperatur zu Nitroaminodiarylverbindungen der Formel

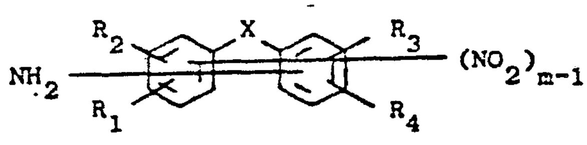

dadurch gekennzeichnet, daß man die Reduktion mit 1-2 Mol Sulfid pro zu reduzierender Nitrogruppe und in Anwesenheit von 0,5-10 Mol Ammoniak pro zu reduzierender Nitrogruppe durchführt.

2. Verfahren nach Anspruch 1, dadurch gekennzeichnet, daß man mit äquivalenten Mengen Sulfid reduziert.

3. Verfahren nach Ansprüchen 1-2, dadurch gekennzeichnet, daß man die Reduktion bei 30-110 °C, vorzugsweise bei 70-90 °C, insbesondere bei 75-85 °C durchführt.

4. Verfahren nach Ansprüchen 1-3, dadurch gekennzeichnet, daß man die Reduktion mit Hydrogensulfiden der Alkalimetalle, Erdalkalimetalle und des Ammoniaks, vorzugsweise mit Natriumhydrogensulfid durchführt.

5. Verfahren nach Ansprüchen 1-4, dadurch gekennzeichnet, daß man 4,4'-Dinitro-stilben-2,2'-disulfonsäure-dinatriumsalz in 4-Nitro-4'-aminostilben-2,2'-disulfonsäure bzw. ihre Salze überführt.

**Claims**

1. Process for the partial reduction of polynitrated diaryl compounds of the formula

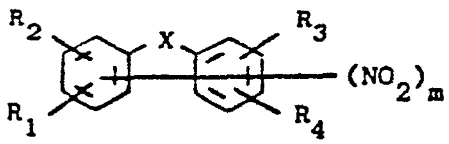

wherein
X = —CH = CH—
$R_1$-$R_4$ independently of one another = hydrogen, $C_1$-$C_4$-alkyl, halogen or sulphonic acid groups and
m = 2-10,
using sulphides or substances which donate sulphide ions, at elevated temperature to give nitro-aminodiaryl compounds of the formula

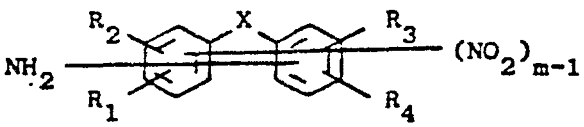

characterised in that the reduction is carried out with 1-2 mols of sulphide per nitro group to be reduced and in the presence of 0.5-10 mols of ammonia per nitro group to be reduced.

2. Process according to Claim 1, characterised in that the reduction is carried out with equivalent amounts of sulphide.

3. Process according to Claims 1-2, characterised in that the reduction is carried out at 30-110 °C, preferably at 70-90 °C and in particular at 75-85 °C.

4. Process according to Claims 1-3, characterised in that the reduction is carried out with bisulphides of the alkali metals, alkaline earth metals and of ammonia, preferably with sodium bisulphide.

5. Process according to Claims 1-4, characterised in that disodium 4,4'-dinitro-stilbene-2,2'-disulphonate is converted into 4-nitro-4'-aminostilbene-2,2'-disulphonic acid or salts thereof.

**Revendications**

1. Procédé de réduction partielle de composés diaryliques polynitrés de formule :

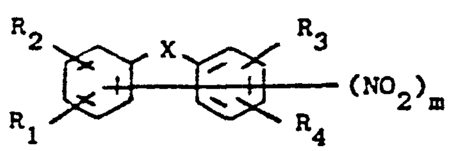

dans laquelle
X = —CH = CH—
$R_1$-$R_4$ représentent indépendamment l'un de l'autre l'hydrogène, des groupes alkyle en $C_1$ à $C_4$, un halogène, des groupes acide sulfonique.
m = 2-10
avec des sulfures ou des substances libérant des ions sulfure à température élevée en composés nitroaminodiaryliques de formule :

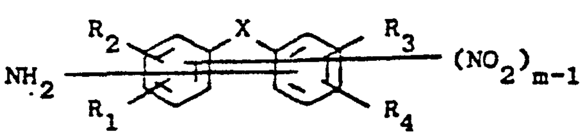

caractérisé en ce qu'on conduit la réduction avec 1 à 2 moles de sulfure par groupe nitro à réduire et en présence de 0,5 à 10 moles d'ammoniac par groupe nitro à réduire.

2. Procédé suivant la revendication 1, caractérisé en ce qu'on conduit la réduction avec des quantités équivalentes de sulfure.

3. Procédé suivant les revendications 1-2, caractérisé en ce qu'on conduit la réduction à 30-110 °C, de préférence à 70-90 °C, notamment à 75-85 °C.

4. Procédé suivant les revendications 1-3, caractérisé en ce qu'on conduit la réaction avec des hydrogénosulfures des métaux alcalins, des métaux alcalino-terreux et de l'ammoniac, de préférence avec l'hydrogéno-sulfure de sodium.

5. Procédé suivant les revendications 1-4, caractérisé en ce qu'on transforme le sel disodique de l'acide 4,4'-dinitrostilbène-2,2'-disulfonique en l'acide 4-nitro-4'-aminostilbène-2,2'-disulfonique ou en ses sels.